(19) Europäisches Patentamt
European Patent Office
Office européen des brevets

(11) Veröffentlichungsnummer: **0 017 080**
**A2**

(12) # EUROPÄISCHE PATENTANMELDUNG

(21) Anmeldenummer: 80101428.3

(22) Anmeldetag: 19.03.80

(51) Int. Cl.³: C 07 D 249/08
C 07 D 233/60, C 07 D 303/08
C 07 D 303/22
//A01N43/64, A01N43/50

(30) Priorität: 28.03.79 DE 2912288

(43) Veröffentlichungstag der Anmeldung:
15.10.80 Patentblatt 80/21

(84) Benannte Vertragsstaaten:
AT BE CH DE FR GB IT NL SE

(71) Anmelder: BAYER AG
Zentralbereich Patente, Marken und Lizenzen
D-5090 Leverkusen 1, Bayerwerk(DE)

(72) Erfinder: Regel, Erik, Ing. Grad.
Bergerheide 72a
D-5600 Wuppertal 1(DE)

(54) **Verfahren zur Herstellung von Hydroxyethyl-azolen; Zwischenprodukte.**

(57) Hydroxyethylazole der Formel

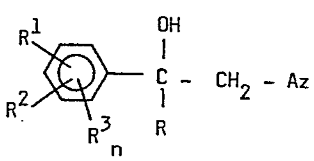

(I)

worin
Az Imidazol oder Triazol und
R gegebenenfalls substituiertes Phenyl, Naphthyl oder Tetrahydronaphthyl bedeuten,

werden nach einem chemisch eigenartigen Verfahren durch Umsetzung von Oxiranen der Formel

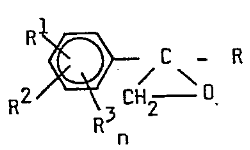

(II)

mit Azolen der Formel

H - Az (III)

in Gegenwart eines Alkalialkoholats und in Gegenwart eines Verdünnungsmittels erhalten.

Die Oxirane der Formel II sind zum Teil neu.

Dieser Teil ist ebenfalls Gegenstand der Erfindung.

EP 0 017 080 A2

BAYER AKTIENGESELLSCHAFT 5090 Leverkusen, Bayerwerk
Zentralbereich
Patente, Marken und Lizenzen Si/AB

27. März 1979

BEZEICHNUNG GEÄNDERT
siehe Titelseite

## Verfahren zur Herstellung von Hydroxyethyl-azolen

Die vorliegende Erfindung betrifft ein neues, chemisch eigenartiges Verfahren zur Herstellung von teilweise bekannten Hydroxyethyl-azolen, welche antimykotische Eigenschaften aufweisen.

Es ist bereits bekannt geworden, daß man bestimmte Hydroxyethyl-imidazole erhält, wenn man entsprechende Hydroxyethyl-halogenide mit Imidazol, gegebenenfalls in Gegenwart eines Säurebindemittels, wie vorzugsweise einem Ueberschuß an Imidazol, oder in Form eines ihrer Alkalisalze, wie sie beispielsweise durch Behandlung mit Natriummethylat in einem geeigneten Lösungsmittel erhalten werden, gegebenenfalls in Gegenwart eines inerten organischen Lösungsmittels, wie z.B. Dimethylformamid, bei Temperaturen zwischen 30 und 120°C nach folgendem Reaktionsschema umsetzt (vergleiche DE-OS 26 23 129):

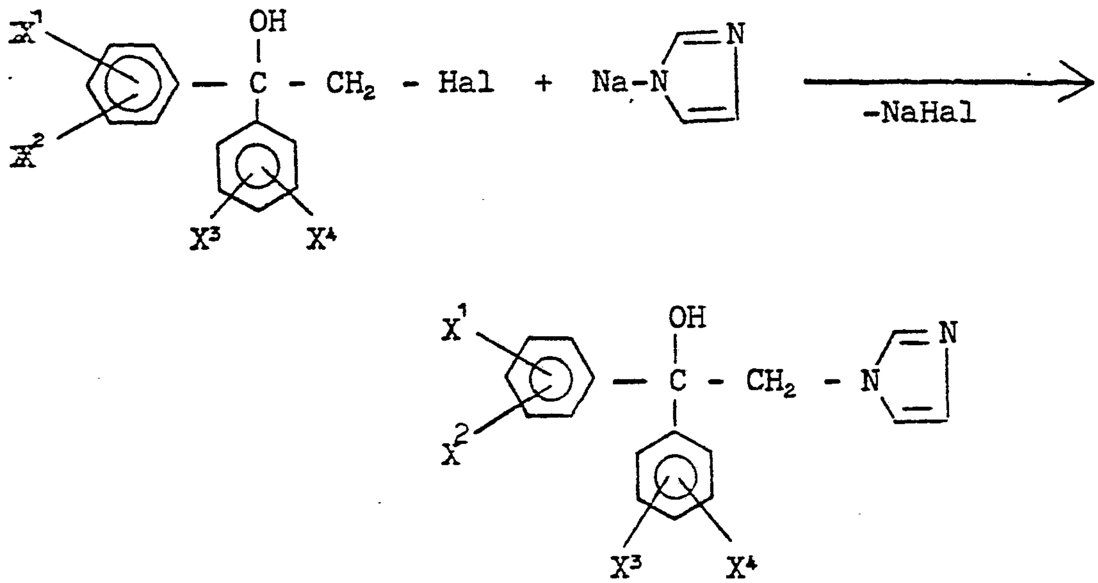


$X^1$ bis $X^4$ = H, Halogen, Alkyl, Alkoxy

Hal = Halogen (Chlor, Brom)

Dieses Verfahren hat den Nachteil, daß es nicht in befriedigenden Ausbeuten abläuft.

Es wurde gefunden, daß man die teilweise bekannten Hydroxyethyl-azole der allgemeinen Formel

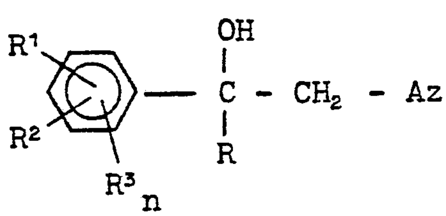
(I)

in welcher

Az für Imidazol oder Triazol steht,

R für gegebenenfalls substituiertes Phenyl, Naphthyl oder Tetrahydronaphthyl steht,

$R^1$ für Wasserstoff, Halogen, Alkyl, Alkoxy, Halogenalkyl, gegebenenfalls substituiertes Phenyl oder Cycloalkyl steht und

$R^2$ für Wasserstoff steht, oder

$R^1$ und $R^2$ gemeinsam in o-Stellung zueinander für eine gegebenenfalls substituierte, mehrgliedrige Methylenbrücke oder zusammen mit dem Phenylring für Naphthyl und gegebenenfalls substituiertes Fluorenyl stehen,

$R^3$ für Halogen, Alkyl, Alkoxy oder Halogenalkyl steht und

n für 0, 1, 2 oder 3 steht,

erhält, wenn man Oxirane der Formel

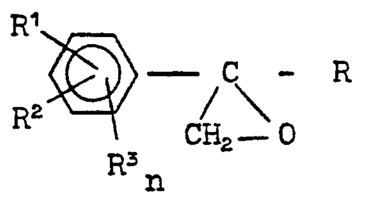

(II)

in welcher

R, $R^1$, $R^2$, $R^3$ und n die oben angegebene Bedeutung haben,

mit Azolen der Formel

H - Az (III)

in welcher

Az die oben angegebene Bedeutung hat,

in Gegenwart eines Alkalialkoholats und in Gegenwart eines Verdünnungsmittels umsetzt.

Es ist als ausgesprochen überraschend zu bezeichnen, daß nach dem erfindungsgemäßen Verfahren die Endprodukte in gewünschter hoher Ausbeute erhalten werden, womit sich dieses Verfahren gegenüber dem aus dem Stand der Technik bekannten als vorteilhafter erweist.

Die nach dem erfindungsgemäßen Verfahren herstellbaren Hydroxylethyl-azole sind durch die Formel (I) allgemein definiert. In dieser Formel steht

<u>Az</u> vorzugsweise für den Imidazol-1-yl-, 1,2,4-Triazolyl-1-yl- und 1,3,4-Triazol-1-yl-Rest;

<u>R</u> steht vorzugsweise für gegebenenfalls substituiertes Phenyl, Naphthyl oder Tetrahydronaphthyl, wobei als Substituenten vorzugsweise genannt seien: Halogen, insbesondere Fluor, Chlor und Brom, geradkettiges oder verzweigtes Alkyl und Alkoxy mit jeweils 1 bis 4 Kohlenstoffatomen, sowie Halogenalkyl mit 1 bis 4 Kohlenstoff- und bis zu 5 Halogenatomen, insbesondere mit 1 oder 2 Kohlenstoff- und bis zu 3 gleichen oder verschiedenen Halogenatomen, wobei als Halogene insbesonder Fluor oder Chlor stehen, beispielsweise sei Trifluormethyl genannt;

$\underline{R^1}$ stent vorzugsweise für Wasserstoff, Halogen, insbesondere Fluor, Chlor und Brom, geradkettiges oder verzweigtes Alkyl und Alkoxy mit jeweils 1 bis 4 Kohlenstoffatomen, sowie Halogenalkyl mit 1 bis 4 Kohlenstoff- und bis zu 5 Halogenatomen, insbesondere mit 1 oder 2 Kohlenstoff- und bis zu 3 gleichen oder verschiedenen Halogenatomen, wobei als Halogene insbesondere Fluor und Chlor stehen, beispielsweise sei Trifluormethyl genannt.

$\underline{R^1}$ steht außerdem vorzugsweise für gegebenenfalls substituiertes Phenyl oder Cycloalkyl mit 3 bis 7 Kohlenstoffatomen, wobei als Substituenten vorzugsweise genannt seien: Halogen, insbesondere Fluor, Chlor oder Brom, sowie Alkyl und Alkoxy mit 1 bis 4, insbesondere mit 1 bis 2 Kohlenstoffatomen, und Nitro.

$\underline{R^2}$ steht vorzugsweise für Wasserstoff; oder

$\underline{R^1 \text{ und } R^2}$ stehen vorzugsweise gemeinsam in ortho-Stellung zueinander für eine gegebenenfalls einfach oder mehrfach substituierte Methylenbrücke mit 3 bis 5 Methylengruppen, wobei als Substituenten vorzugsweise genannt seien: Halogen, insbesondere Fluor, Chlor oder Brom, sowie Alkyl mit 1 bis 4, insbesondere mit 1 bis 2 Kohlenstoffatomen.

$\underline{R^1 \text{ und } R^2}$ stehen außerdem zusammen mit dem Phenylring, an den sie gebunden sind, für Naphthyl sowie für Fluorenyl, das gegebenenfalls durch Halogen oder Alkyl mit 1 bis 2 Kohlenstoffatomen substituiert sein kann.

$R^3$ steht vorzugsweise für Halogen, insbesondere Fluor, Chlor oder Brom, geradkettiges oder verzweigtes Alkyl und Alkoxy mit jeweils 1 bis 4 Kohlenstoffatomen, sowie für Halogenalkyl mit 1 bis 4 Kohlenstoffatomen und bis zu 5 Halogenatomen, insbesondere mit 1 oder 2 Kohlenstoffatomen und bis zu 3 gleichen oder verschiedenen Halogenatomen, wobei als Halogene insbesondere Fluor und Chlor stehen, beispielsweise sei Trifluormethyl genannt;

n steht vorzugsweise für die Zahlen 0, 1 oder 2.

Ganz besonders bevorzugt sind diejenigen Verbindungen der Formel (I), in denen Az für Imidazol-1-yl oder 1,2,4-Triazol-1-yl steht; R für Phenyl, das gegebenenfalls einfach oder zweifach durch Chlor, Fluor oder Methyl substituiert ist, für Naphthyl und Tetrahydronaphthyl steht; $R^1$ für Wasserstoff, Fluor, Chlor, Brom, Methyl, Ethyl, Methoxy, Ethoxy, Trifluormethyl, ferner für Phenyl, Cyclopentyl oder Cyclohexyl steht, die gegebenenfalls einfach oder zweifach durch Chlor, Brom, Fluor, Nitro, Methyl oder Methoxy substituiert sein können; und $R^2$ für Wasserstoff steht, oder $R^1$ und $R^2$ gemeinsam in ortho-Stellung zueinander für eine Tri-, Tetra- oder Pentamethylenbrücke stehen, die gegebenenfalls durch Chlor oder Methyl substituiert sind, oder zusammen mit dem Phenylring, an den sie gebunden sind, für Naphthyl oder gegebenenfalls durch Chlor oder Methyl substituiertes Fluorenyl stehen; $R^3$ für Chlor, Fluor oder Methyl steht und n für 0 oder 1 steht.

Verwendet man beispielsweise 2-(4-Biphenylyl)-2-(2-chlorphenyl)-oxiran, Imidazol und Natriummethylat als Ausgangsstoffe, so kann der Reaktionsablauf durch das folgende Formelschema wiedergegeben werden:

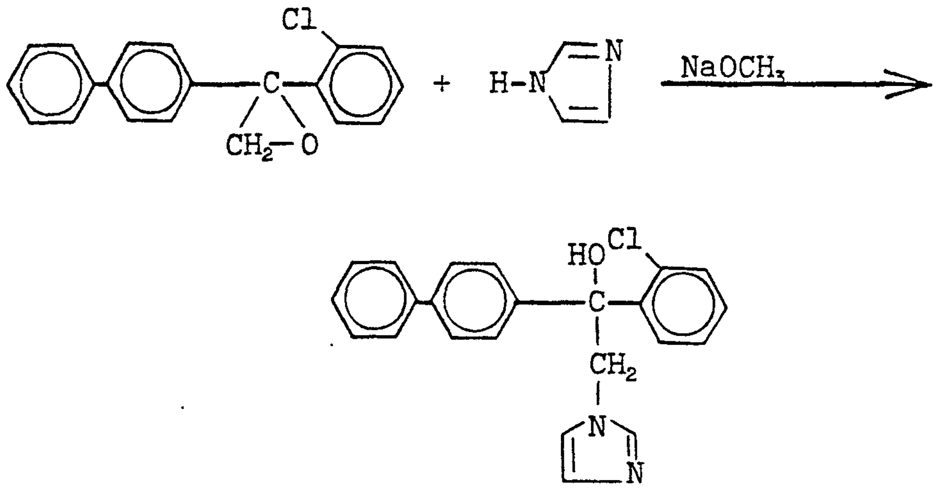


Die bei der Durchführung des erfindungsgemäßen Verfahrens als Ausgangsstoffe zu verwendenden Oxirane sind durch die Formel (II) allgemein definiert. In dieser Formel stehen R, $R^1$, $R^2$, $R^3$ und n vorzugsweise für die Reste, die bereits im Zusammenhang mit der Beschreibung der erfindungsgemäßen Stoffe der Formel (I) vorzugsweise für diese Substituenten genannt wurden.

Die Oxirane der Formel (II) sind teilweise bekannt (vergleiche u.a. DE-OS 24 08 658)

Noch nicht bekannt sind die Oxirane der Formel

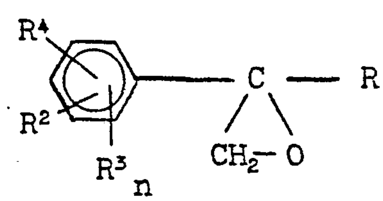

(IV)

in welcher

R für gegebenenfalls substituiertes Phenyl, Naphthyl oder Tetrahydronaphthyl steht,

$R^1$ für gegebenenfalls substituiertes Phenyl oder Cycloalkyl steht und

$R^2$ für Wasserstoff steht oder

$R^2$ und $R^1$ gemeinsam in o-Stellung zueinander für eine gegebenenfalls substituierte, mehrgliedrige Methylenbrücke oder zusammen mit dem Phenylring für Naphthyl und gegebenenfalls substituiertes Fluorenyl stehen,

$R^3$ für Halogen, Alkyl, Alkoxy oder Halogenalkyl steht und

n für 0, 1, 2 oder 3 steht.

Die Oxirane der Formel (II) werden erhalten, indem man entsprechende Ketone der Formel

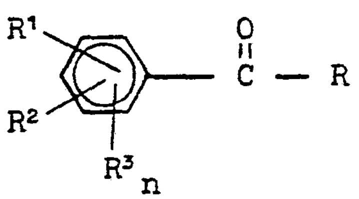

(V)

in welcher

R, $R^1$, $R^2$, $R^3$ und n die oben angegebene Bedeutung haben,

α) mit Dimethyloxosulfonium-methylid der Formel

$$(CH_3)_2\overset{\oplus}{S}O\overset{\ominus}{C}H_3 \qquad (VI)$$

in an sich bekannter Weise in Gegenwart eines Verdünnungsmittels, wie beispielsweise Dimethylsulfoxid, bei Temperaturen zwischen 20 und 80°C umsetzt (vergleiche hierzu auch die Angaben in JACS <u>87</u>, 1353-1364 (1965)), oder

ß) mit Trimethylsulfonium-methylsulfat der Formel

$$(CH_3)_3\overset{\oplus}{S}CH_3SO_4^{\ominus} \qquad (VII)$$

in an sich bekannter Weise in Gegenwart eines Zweiphasensystems und gegebenenfalls in Gegenwart eines Phasentransferkatalysators bei Temperaturen zwischen 0 und 100°C umsetzt (vergleiche auch die Angaben in Heterocycles <u>8</u>, 397 (1977)).

Die Ketone der Formel (V) sind allgemein bekannte Verbindungen der organischen Chemie.

Das Dimethyloxosulfonium-methylid der Formel (VI) ist ebenfalls bekannt und wird in situ eingesetzt, indem man Trimethyloxosulfoniumjodid mit Natriumhydrid (vergleiche auch die o.g. Literaturstelle) bzw. Natriumamid umsetzt. Das Trimethylsulfonium-methylsulfat ist auch bekannt und wird in situ eingesetzt, indem man Dimethylsulfid mit Dimethylsulfat umsetzt (vergleiche auch die o.g. Literaturstelle).

Als Beispiele der Oxirane der Formel (II) seien genannt:

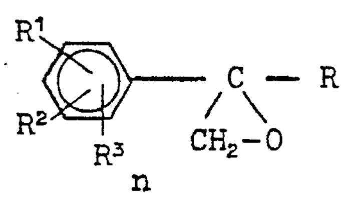

(II)

| R | $R^1$ | $R^2$ | $R^3{}_n$ |
|---|---|---|---|
| 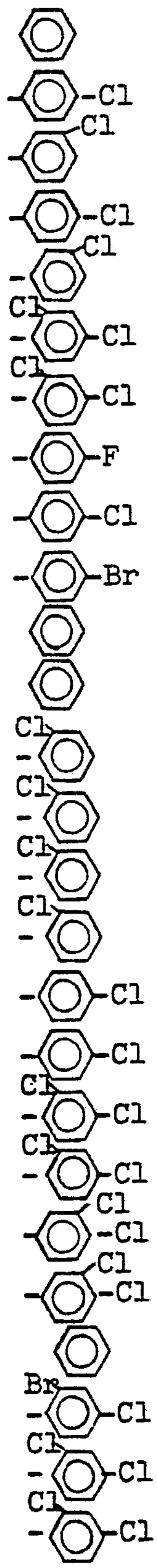 | H | H | - |
| | 4-Cl | H | - |
| | 3-Cl | H | - |
| | 4-Cl | H | 2-Cl |
| | 4-Cl | H | 2-Cl |
| | 4-Cl | H | 2-Cl |
| | 2-Cl | H | 6-Cl |
| | 2-Cl | H | 4-Cl |
| | H | H | - |
| | H | H | - |
| | 2-Cl | H | 4-Cl |
| | 2-Br | H | 4-Cl |
| | H | H | - |
| | 2-Cl | H | 4-Cl |
| | 2-Br | H | 4-Cl |
| | $2-CH_3$ | H | 4-Cl |
| | 4-Br | H | - |
| | 2-Br | H | 4-Cl |
| | 2-Br | H | 4-Cl |
| | $4-OCH_3$ | H | - |
| | $4-OCH_3$ | H | - |
| | 4-Cl | H | 2-Br |
| | 2-Cl | H | 6-Cl |
| | 2-Cl | H | 6-Cl |
| | $4-CH_3$ | H | - |
| | 4-Cl | H | $2-CH_3$ |

| R | $R^1$ | $R^2$ | $R^3{}_n$ |
|---|---|---|---|
| 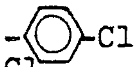 | H | H | 4-$OCH_3$ |
| 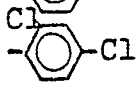 | 4-F | H | – |
| 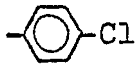 | 4-Cl | H | 2-$CH_3$ |
| 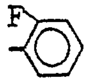 | 2-Cl | H | – |
| 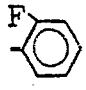 | 4-Cl | H | – |
| 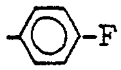 | 4- 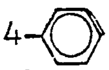 | H | – |
| 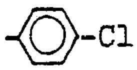 | 4- 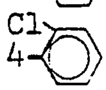 | H | – |
| 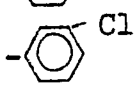 | 4- 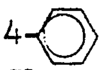 | H | – |
| 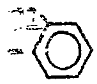 | 4- 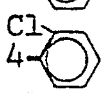 | H | – |
| 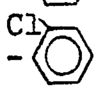 | 4- 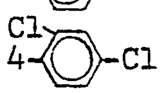 | H | – |
| 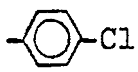 | 4- 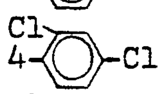 | H | – |
| 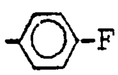 | 4- 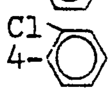 | H | – |
| 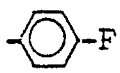 | 4- 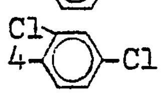 | H | – |
| 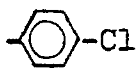 | 4- 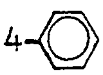 | H | – |
| 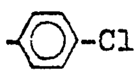 | 4- 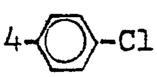 | H | – |
| 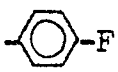 | 4- 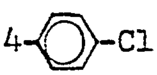 | H | – |
| 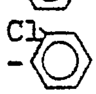 | 4- 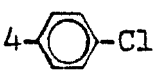 | H | – |
| 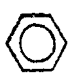 | 4- 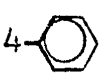 | H | – |
| 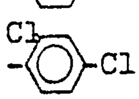 | 4- 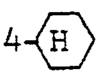 | H | – |
| 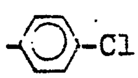 | 3,4-$(CH_2)_3$- | | – |

| R | $R^1$ | $R^2$ | $R^3{}_n$ |
|---|---|---|---|
| 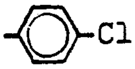 | 3,4-$(CH_2)_4$- | | - |
| 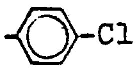 | 3,4 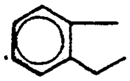 | | - |
| 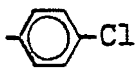 | 3,4- 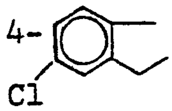 | | - |
| 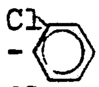 | 4- 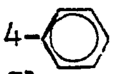 | H | - |
| 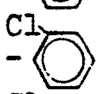 | 4- 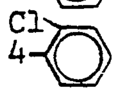 | H | - |
| 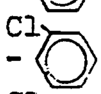 | 4- 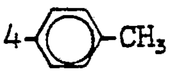 | H | - |
| 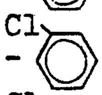 | 4- 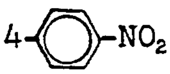 | H | - |
| 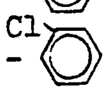 | 4- 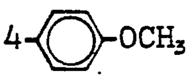 | H | - |
| 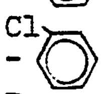 | 4- 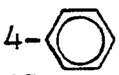 | H | - |
| 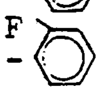 | 4- 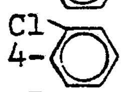 | H | - |
| 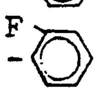 | 4- 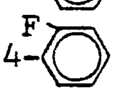 | H | - |
| 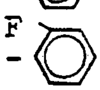 | 4- 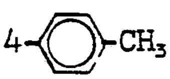 | H | - |
| 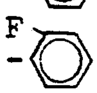 | 4- 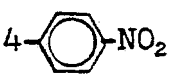 | H | - |
| 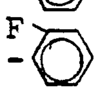 | 4- 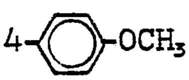 | H | - |

Die erfindungsgemäße Umsetzung wird in Gegenwart eines Alkalialkoholats durchgeführt. Hierzu gehören vorzugsweise die Methylate und Ethylate von Natrium und Kalium.

Für die erfindungsgemäße Umsetzung kommen als Verdünnungsmittel vorzugsweise inerte organische Lösungsmittel infrage. Hierzu gehören vorzugsweise Nitrile, wie insbesondere Acetonitril; aromatische Kohlenwasserstoffe, wie Benzol, Toluol und Dichlorbenzol; Formamide, wie insbesondere Dimethylformamid; halogenierte Kohlenwasserstoffe, wie Methylenchlorid oder Tetrachlorkohlenstoff; sowie Hexamethylphosphortriamid.

Die Reaktionstemperaturen können bei der Durchführung des erfindungsgemäßen Verfahrens in einem größeren Bereich variiert werden. Im allgemeinen arbeitet man zwischen etwa 30 bis 150°C, vorzugsweise zwischen 30°C und 100°C.

Bei der Durchführung des erfindungsgemäßen Verfahrens setzt man auf 1 Mol Oxiran der Formel (II) vorzugsweise 1 bis 3 Mol Azol der Formel (III) und 1 bis 2 Mol Alkalialkoholat ein. Die Isolierung der Endprodukte erfolgt in allgemein üblicher Weise.

In einer bevorzugten Ausführungsform werden die gemäß Verfahren (α) oder (ß) erhaltenen Oxirane der Formel (II) ohne Isolierung direkt weiter umgesetzt (vergleiche die Herstellungsbeispiele).

Die erfindungsgemäß herstellbaren Wirkstoffe der Formel(I) zeichnen sich durch gute antimykotische Wirksamkeit aus (vergleiche DE-OS 26 23 129 sowie die deutschen Patentanmeldungen P 28 51 143 [LeA 19 270] vom 25.11.1978 und P 28 51 116[LeA 19 273] vom 25.11.1978).

Das erfindungsgemäße Verfahren sei anhand der folgenden Herstellungsbeispiele erläutert.

Herstellungsbeispiele

Beispiel 1

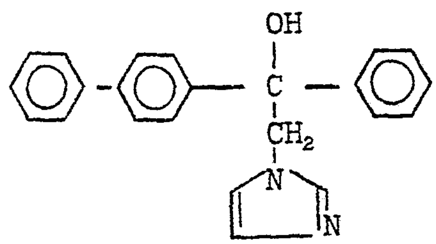

(erfindungsgemäßes Verfahren ohne Isolierung des Zwischenproduktes)

3,6g (0,12 Mol) 80%-iges Natriumhydrid und 26,4g (0,12 Mol) Trimethyloxosulfoniumjodid werden innerhalb 20 Minuten mit 120 ml Dimethylsulfoxid versetzt. Nach beendeter Wasserstoffentwicklung wird eine Lösung von 25,8g (0,1 Mol) 4-Biphenylyl-phenyl-keton in 150 ml Dimethylsulfoxid zugetropft und 2 Stunden bei 60°C nachgerührt. Das erhaltene Reaktionsgemisch wird tropfenweise mit 300 ml Wasser versetzt. Das resultierende Oel wird von der wässrigen Phase abgetrennt, in Chloroform gelöst, die Lösung mit Wasser gewaschen, über Natriumsulfat getrocknet und durch Abdestillieren des Lösungsmittels im Vakuum eingeengt. Das so erhaltene rohe 2-(4-Biphenylyl)-2-phenyloxiran wird in 150 ml Dimethylformamid gelöst. Diese Lösung tropft man zu einer Mischung aus 7g (0,13 Mol) Natriummethylat, 40ml Methanol und 15g (0,22 Mol) Imidazol. Das Reaktionsgemisch wird 4 Stunden auf 80°C erhitzt. Danach wird das Reaktionsgemisch im Vakuum eingeengt und auf Wasser gegossen, die entstehenden Kristalle werden abfiltriert und mit Acetonitril gewaschen. Man erhält 26,7g (83,5 % der Theorie) 1-(4-Biphenylyl)-2-(imidazol-1-yl)-1-phenyl-ethanol vom Schmelzpunkt 224°C.

(bekanntes Verfahren )

2,45g (0,045 Mol) Natriummethylat werden in 20 ml Methanol gelöst und mit 5,25g (0,077 Mol) Imidazol versetzt. Dazu wird eine Lösung von 11,1g (0,077 Mol) 1-(4-Biphenylyl)-2-chlor-1-phenyl-ethanol in 50 ml Dimethylformamid getropft und das Reaktionsgemisch 16 Stunden unter Rückfluß erhitzt. Danach wird das Reaktionsgemisch auf Wasser gegossen und die entstehenden Kristalle werden mit heißem Acetonitril extrahiert. Man erhält 3,2g (21 % der Theorie) 1-(4-Biphenylyl)-2-(imidazol-1-yl)-1-phenyl-ethanol vom Schmelzpunkt 224°C.

Herstellung des Ausgangsproduktes

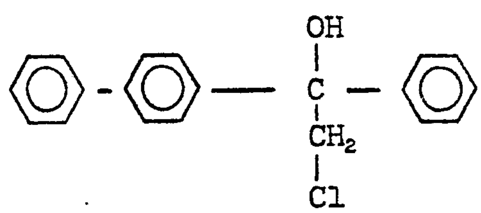

Zu einer Lösung von Phenylmagnesiumbromid, erhalten aus 4,86g(0,2 Mol) Magnesium und 21g (0,2 Mol) Brombenzol in 120 ml Ether, werden 23g (0,1 Mol) 4-Phenyl-phenacylchlorid portionsweise zugegeben. Nach einstündigem Rühren wird das Reaktionsgemisch auf wässrige Ammoniumchlorid -Lösung gegossen. Die abgetrennte Etherphase wird mit Wasser gewaschen, über Natriumsulfat getrocknet und eingeengt. Man erhält 11,1g (36 % der Theorie) 1-(4-Biphenylyl)-2-chlor-1-phenyl-ethanol vom Schmelzpunkt 94°C.

Beispiel 2

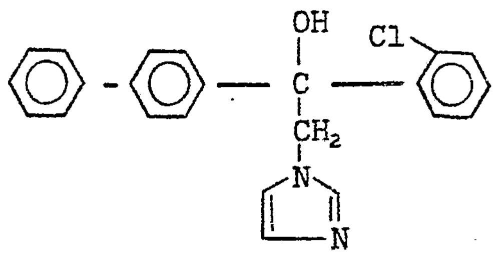

(erfindungsgemäßes Verfahren mit Isolierung des Zwischenproduktes)

3,5g (0,065 Mol) Natriummethylat werden in 20 ml Methanol gelöst und mit 7,5g (0,11 Mol) Imidazol versetzt. Dazu wird eine Lösung von 15g (0,05 Mol) 2-(4-Biphenylyl)-2-(2-chlorphenyl)-oxiran in 75 ml Dimethylformamid getropft und das Reaktionsgemisch 1,5 Stunden auf 80°C erhitzt. Danch wird das Reaktionsgemisch auf Wasser gegossen, die entstehenden Kristalle werden abfiltriert und getrocknet. Man erhält 14,7 g (79 % der Theorie) 1-(4-Biphenylyl)-1-(2-chlorphenyl)-2-imidazol-1-yl-ethanol vom Schmelzpunkt 222°C.

Herstellung des Ausgangsproduktes

(neues Zwischenprodukt der Formel (IV))

(IV-1)

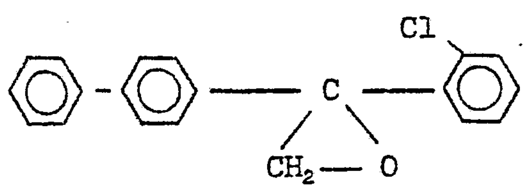

2,7g (0,09 Mol) 80%-iges Natriumhydrid und 19,8 g(0,09 Mol) Trimethyloxosulfoniumjodid werden innerhalb 20 Minuten mit 90 ml Dimethylsulfoxid versetzt. Nach beendeter Wasserstoffentwicklung wird eine Lösung von 22g (0,075 Mol) 2-Chlor-4'-phenyl-benzophenon in 60 ml Dimethylsulfoxid zugetropft und 1 Stunde bei 50°C nachgerührt. Das

erkaltete Reaktionsgemisch wird mit 200 ml Wasser versetzt und mit Ether ausgeschüttelt. Die Etherlösung wird mit Wasser gewaschen, über Natriumsulfat getrocknet und durch Abdestillieren des Lösungsmittels in Vakuum eingeengt. Der Rückstand wird aus Diisopropylether umkristallisiert. Man erhält 15g (65 % der Theorie) 2-(4-Biphenylyl)-2-(2-chlorphenyl)-oxiran vom Schmelzpunkt 70°C.

In entsprechender Weise können die in der folgenden Tabelle 1 aufgeführten Verbindungen der Formel (I) erhalten werden:

<u>T a b e l l e   1</u>

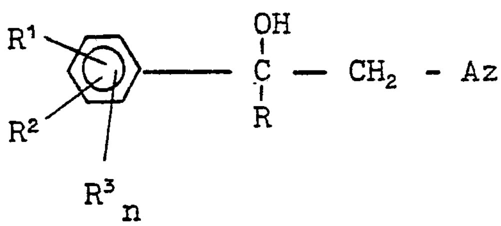 (I)

| Bsp. Nr. | R | $R^1$ | $R^2$ | $R^3_n$ | Az | Schmelzpunkt (°C) |
|---|---|---|---|---|---|---|
| 3 | 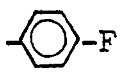 | 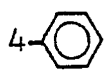 | H | - | 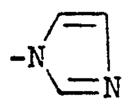 | 220 |
| 4 | 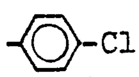 | 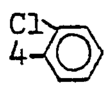 | H | - | 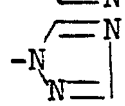 | 190 |
| 5 | 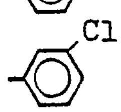 | 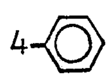 | H | - | 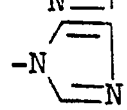 | |
| 6 | 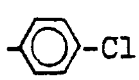 | 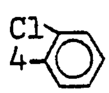 | H | - | 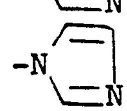 | 187 |
| 7 | 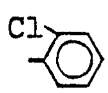 | 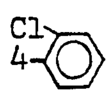 | H | - | 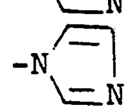 | 206 |
| 8 | 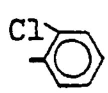 | 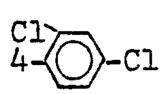 | H | - | 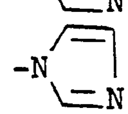 | 220 |

| Bsp. Nr. | R | $R^1$ | $R^2$ | $R^3_n$ | Az | Schmelzpunkt (°C) |
|---|---|---|---|---|---|---|
| 9 | 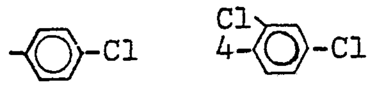 | | H | - | 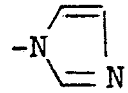 | 176 |
| 10 | 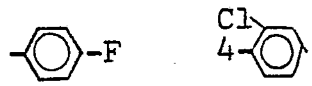 | | H | - | 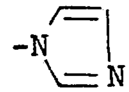 | 100 |
| 11 | 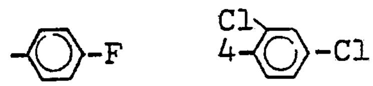 | | H | - | 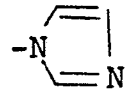 | 225 |
| 12 | 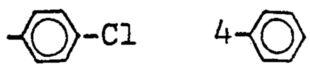 | | H | - | 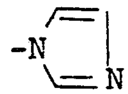 | 230 |
| 13 | 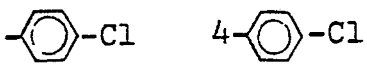 | | H | - | 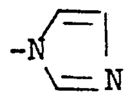 | 188 |
| 14 | 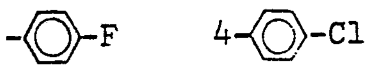 | | H | - | 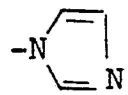 | 218 |
| 15 | 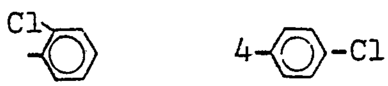 | | H | - | 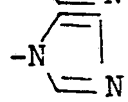 | 206 |
| 16 | 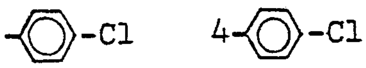 | | H | - | 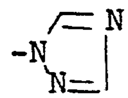 | 189 |
| 17 | 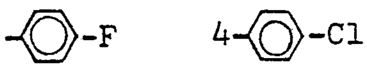 | | H | - | 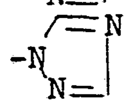 | 217 |
| 18 | 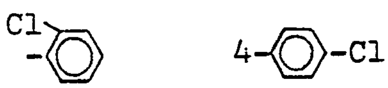 | | H | - | 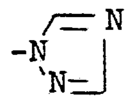 | 144 |
| 19 | 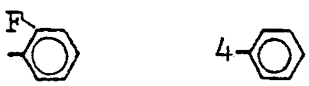 | | H | - | 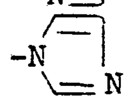 | 202 |
| 20 | 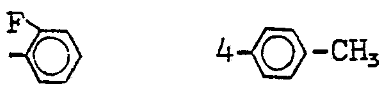 | | H | - | 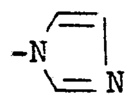 | 200 |
| 21 | 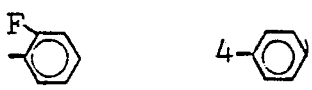 | | H | - | 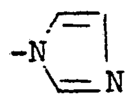 | 164 |
| 22 | 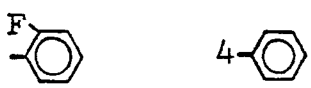 | | H | - | 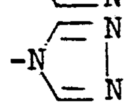 | 170 |
| 23 | 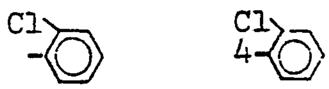 | | H | - | 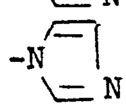 | 206 |
| 24 | 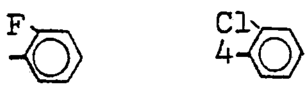 | | H | - | 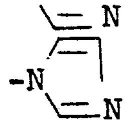 | 227 |
| 25 | 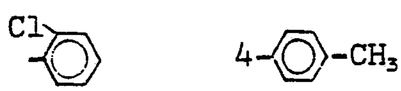 | | H | - | 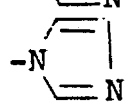 | 207 |
| 26 | 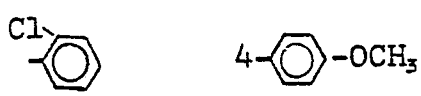 | | H | - | 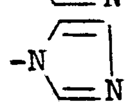 | |

| Bsp. Nr. | R | $R^1$ | $R^2$ | $R^3{}_n$ | Az | Schmelz-punkt (°C) |
|---|---|---|---|---|---|---|
| 27 | 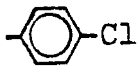 | 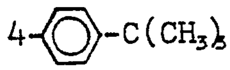 | H | - | 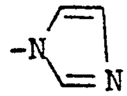 | |
| 28 | 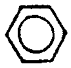 | H | H | - | 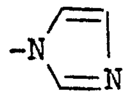 | 208-10 |
| 29 | 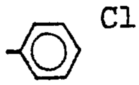 | 3-Cl | H | - | 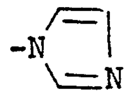 | 165 |
| 30 | 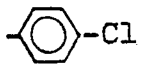 | 4-Cl | H | - | 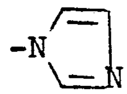 | 200 |
| 31 | 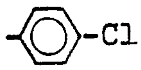 | 2-Cl | H | 4-Cl | 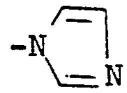 | 217-18 ($xHNO_3$) |
| 32 | 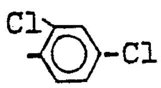 | 3-Cl | H | - | 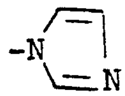 | 252 |
| 33 | 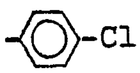 | 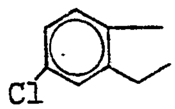 | | - | 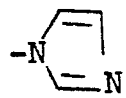 | 242 |
| 34 | 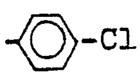 | 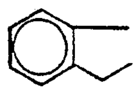 | | - | 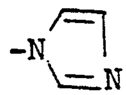 | 238 |
| 35 | 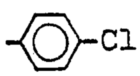 | 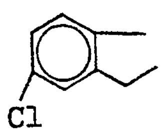 | | - | 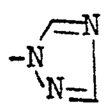 | 186 |

Ensprechend Beispiel 1 und 2 können die in der folgenden Tabelle aufgeführten neuen Zwischenprodukte der Formel (IV) erhalten werden:

Tabelle 2

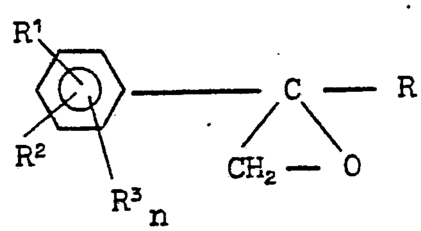

(II)

| Bsp. Nr. | R | $R^1$ | $R^2$ | $R^3{}_n$ | Schmelz-punkt(°C) |
|---|---|---|---|---|---|
| (IV-2) | 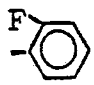 | 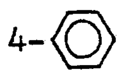 | H | - | 89 |
| (IV-3) | 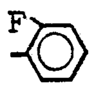 | 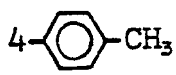 | H | - | 109 |
| (IV-4) | 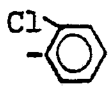 | 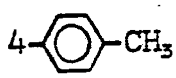 | H | - | Oel |
| (IV-5) | 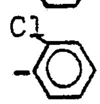 | 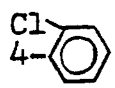 | H | - | Oel |
| (IV-6) | 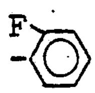 | 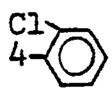 | H | - | Oel |
| (IV-7) | 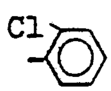 | 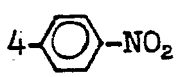 | H | - | |
| (IV-8) | 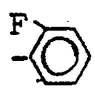 | 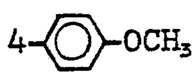 | H | - | |

Patentansprüche

1. Verfahren zur Herstellung von Hydroxyethylazolen der Formel

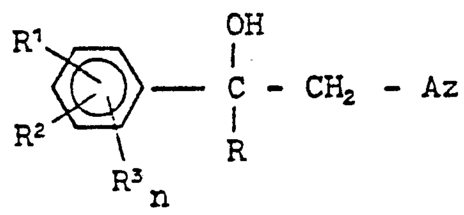

(I)

in welcher

Az für Imidazol oder Triazol steht,

R für gegebenenfalls substituiertes Phenyl, Naphthyl oder Tetrahydronaphthyl steht,

$R^1$ für Wasserstoff, Halogen, Alkyl, Alkoxy, Halogenalkyl, gegebenenfalls substituiertes Phenyl oder Cycloalkyl steht und

$R^2$ für Wasserstoff steht, oder

$R^1$ und $R^2$ gemeinsam in o-Stellung zueinander für eine gegebenenfalls substituierte, mehrgliedrige Methylenbrücke oder zusammen mit dem Phenylring für Naphthyl und gegebenenfalls substituiertes Fluorenyl stehen,

$R^3$ für Halogen, Alkyl, Alkoxy oder Halogenalkyl steht und

n für 0, 1, 2 oder 3 steht,

dadurch gekennzeichnet, daß man Oxirane der Formel

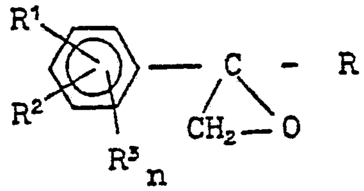

(II)

in welcher

$R$, $R^1$, $R^2$, $R^3$ und $n$ die oben angegebene Bedeutung haben,

mit Azolen der Formel

H - Az (III)

in welcher

Az die oben angegebene Bedeutung hat,

in Gegenwart eines Alkalialkoholats und in Gegenwart eines Verdünnungsmittels umsetzt.

2. Verfahren nach Anspruch 1, dadurch gekennzeichnet, daß man solche Oxirane der Formel (II) umsetzt, bei denen

<u>Az</u> für Imidazol-1-yl oder 1,2,4-Triazol-1-yl steht;

<u>R</u> für Phenyl, das gegebenenfalls einfach oder zweifach durch Chlor, Fluor oder Methyl substituiert ist, für

Naphthyl und Tetrahydronaphthyl steht;

$R^1$ für Wasserstoff, Fluor, Chlor, Brom, Methyl, Ethyl, Methoxy, Ethoxy, Trifluormethyl, ferner für Phenyl, Cyclopentyl oder Cyclohexyl steht, die gegebenenfalls einfach oder zweifach durch Chlor, Brom, Fluor, Nitro, Methyl oder Methoxy substituiert sein können;

$R^2$ für Wasserstoff steht, oder

$R^1$ und $R^2$ gemeinsam in ortho-Stellung zueinander für eine Tri-, Tetra- oder Pentamethylenbrücke stehen, die gegebenenfalls durch Chlor oder Methyl substituiert sind, oder zusammen mit dem Phenylring, an den sie gebunden sind, für Naphthyl oder gegebenenfalls durch Chlor oder Methyl substituiertes Fluorenyl stehen;

$R^3$ für Chlor, Fluor oder Methyl stehen und

n für 0 oder 1 steht.

3. Verfahren nach Anspruch 1, dadurch gekennzeichnet, daß man die Umsetzung bei Temperaturen von 30 bis 150°C, vorzugsweise 30 bis 100°C durchführt.

4. Verfahren nach Anspruch 1, dadurch gekennzeichnet, daß man auf 1 Mol Oxiran der Formel (II) 1 bis 3 Mol Azol der Formel (III) und 1 bis 2 Mol Alkalialkoholat einsetzt.

5. Oxirane der Formel

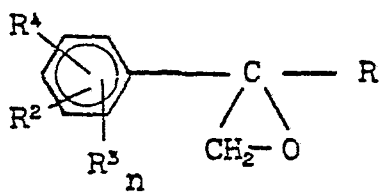

(IV)

in welcher

R für gegebenenfalls substituiertes Phenyl, Naphthyl oder Tetrahydronaphthyl steht,

$R^1$ für gegebenenfalls substituiertes Phenyl oder Cycloalkyl steht und

$R^2$ für Wasserstoff steht oder

$R^2$ und $R^1$ gemeinsam in o-Stellung zueinander für eine gegebenenfalls substituierte, mehrgliedrige Methylenbrücke oder zusammen mit dem Phenylring für Naphthyl und gegebenenfalls substituiertes Fluorenyl stehen,

$R^3$ für Halogen, Alkyl, Alkoxy oder Halogenalkyl steht und

n für 0, 1, 2 oder 3 steht.